# EUROPEAN PATENT SPECIFICATION

(11) **EP 2 488 133 B1**
(45) Date of publication and mention of the grant of the patent: **06.08.2014**
(21) Application number: 10763638.3
(22) Date of filing: 22.09.2010
(51) Int. Cl.: A61F 5/41, A61F 5/042

(54) **Device for shaping a body part**
Vorrichtung zum Formen eines Körperteils
Dispositif de mise en forme d'une partie du corps

(30) Priority: 15.10.2009 EP 09173109
(43) Date of publication of application: 22.08.2012
(73) Proprietor: Assink, Loek Antonius Bernardus, 1111 HN Diemen (NL)
(72) Inventor: Assink, Loek Antonius Bernardus, 1111 HN Diemen (NL)
(74) Representative: De Vries & Metman
(86) International application number: PCT/EP2010/063942
(87) International publication number: WO 2011/045151

(56) References cited:
- EP-A1- 0 370 932
- CA-A1- 2 132 552
- CA-A1- 2 444 663
- CN-Y- 201 260 711
- RU-C1- 2 014 044
- SU-A1- 1 725 878

## Description

### TECHNICAL FIELD

The present invention relates to a device for shaping a body part, in particular enlarging an elongate body part, such as a human penis, by applying a force to the body part to be shaped.

### BACKGROUND

Throughout history humans have sought ways to adorn their bodies by shaping of one or more body parts, such as by extending protruding and/or elongate body parts. In particular, men have sought ways to increase the size of their penises, most notably their length. The predominant technique has been suspending one or more weights from the body part and using the pull of gravity to obtain a desired lengthening.

For extending a penis devices based on vacuum-suction have been developed. Such devices are generally bulky, complicated and expensive and require attention during use. Thus, such devices cannot be worn inconspicuous and/or for prolonged times.

Spring based devices for extending a penis are also known. The type of WO 96/26691, WO 97/28764, WO 2006/058398, US 2007/0179337 and US 7,578,785 generally comprise a fixture with a number of spring-loaded rigid rods extending between a ring-shaped base portion for surrounding the penis root and abutting a body portion, e.g. the pelvic bone, and a fastening portion for releasable fastening to and pulling on the glans penis. During use the spring-loaded rods extend along the penis and a traction force is applied to the penis. The rigid rods are formed telescopic and comprise screw-threads, so that the length of the rods and the applied tension force are adjustable.

Such spring based devices are complicated to adjust for length. Further, in use, the penis is held substantially perpendicular to the body of the user, e.g. to the pelvic bone, which is quite conspicuous and which hinders movement. Treatment is therefore generally restricted to short periods in discrete surroundings.

WO 2007/019687 discloses a method and a device to provide penis enlargement. The device is to be attached to an article of clothing, a body part or an inanimate object. The tension applied to the penis is dependent of body position.

In US 6,416,460 a penis extension device is disclosed which is based on an elastic belt surrounding the body. This pulls the penis in an unnatural and uncomfortable position which may be conspicuous and which may lead to an asymmetric (growth of the) penis.

Further, CA 2 132 552 discloses a penis augmentation device using the force of a compression-spring appliance. The spring is cone shaped and calibrated to penile size. The spring comprises on its upper-most ring tangs. The penis is to be taped and secured to the tangs by a fabric adhesive. Tension and length of the device are selected by adjusting the length of two elastic tension bands secured to windings the spring by Velcro® (hook-and-loop-type fastener). The elasticity of the tension bands of this device affects the spring constant of the device, which therefore becomes difficult to predict and/or adjust and which makes that the effective length of the device cannot be reliably adjusted. Moreover, hook-and-loop type fasteners are known to suddenly and unexpectedly yield and become separated under continuous tension, which in this case could lead to serious pain and injury.

Thus, there is a desire for an improved device for shaping an elongate body part, in particular extending a penis.

It is further noted that DE 523 141 discloses a rubber support sleeve for a penis. In use, the support sleeve is fit around the penis to straighten it in order to mimic an erect penis.

### SUMMARY

Claim 1 provides an improved device. The device is configured to provide a (e.g. tension) force to the body part to shape, e.g. elongate, it by action of the spring. The spring accommodates the body part and protects the body part from outside forces, which body part may have become sensitive by the treatment. The coupling means being attachable to the spring in at least two positions allows reliable determination of the length of the device with respect to a relaxed spring (not compressed or extended). The device of claim 2 further facilitates donning the device and, in particular in the device of claim 3, facilitates selecting or adjusting the position of the coupling means. Thus the length and the spring force of the device in operation are reliably predictable and/or adjustable to a desired value. Moreover, operative safety of the device is improved.

Preferably the spring is configured for accommodating at least a portion of a human penis. However, the device may be configured for accommodating at least a portion of a leg, an arm, a finger or any other elongate body part to be shaped e.g. after injury, operation and/or to correct asymmetric growth.

The coupling means may be adjustable to accommodate body parts to be shaped, e.g. extended, of different sizes, facilitating manufacture and sale. In a particularly cost-efficient embodiment, the spring comprises a wound coil and the coupling means comprise an elastic band which may be loop-shaped.

These and other aspects of the invention and further benefits will hereafter be more fully explained with reference to the drawings.

### BRIEF DESCRIPTION OF THE DRAWINGS

The figures of the drawings show embodiments of the invention by way of example. It is noted that the figures are schematic, not necessarily to scale and that details that are not required for understanding the present invention may have been omitted. The terms "upward", "downward", "below", "above", and the like relate to the embodiments as oriented in the figures. Further, elements that are at least substantially identical or that perform an at least substantially identical function are denoted by the same numeral.
Figs. 1A and 1B show a first embodiment of a penis extender in two arrangements;
Fig. 2 shows a second embodiment of a penis extender;
Fig. 3 shows detail III of Fig. 2;
Fig. 4 illustrates a portion of a different type spring;
Fig. 5 illustrates coupling means of another embodiment.

### DETAILED DESCRIPTION OF EMBODIMENTS

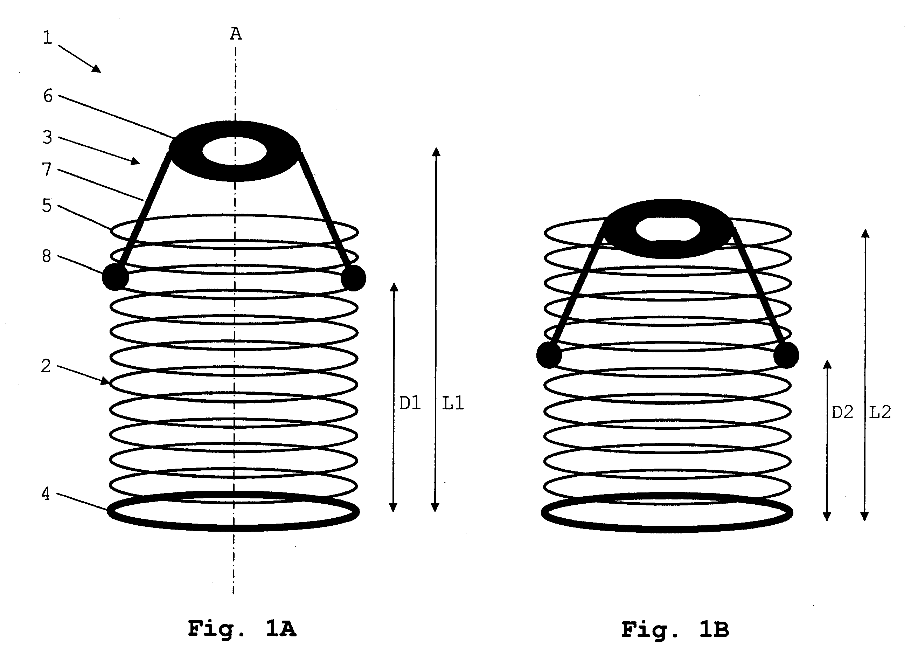

Figs. 1A and 1B show a penis extender 1 comprising a coiled spring 2 and a coupling means 3. The spring 2 has a proximal end 4 and a distal end 5. The spring 2 is substantially cylindrical and extends along an axis of extension A. The spring 2 further has a free inner diameter of sufficient size to accommodate the body part to be extended, here a penis. The coupling means 3 comprise a coupling portion 6 for coupling to the penis and connecting portions 7 for attachment to the spring 2. The coupling portion 6 comprises means such as one or more a soft tissue and/or elastic straps for encircling the penis in a tight-fitting arrangement at a distal portion, e.g. at or near the corona or the glans penis, and holding the penis. The connecting portions 7 are releasably attached to a winding of the spring 2 with connectors 8, e.g. comprising a bolt and a wing nut for clamping to the winding. From comparing Figs. 1A and 1B it will be evident that the coupling means 3 are attachable to the spring 2 at a first position at a first distance D1 from the proximal end 4 of the spring 2, at a different second position at a second distance D2 from the proximal end or any other desired position along the spring 2. Thus, a first effective length L1 and a second effective length L2 or any other desired effective length may be provided. A coiled spring allows attachment in arbitrary positions along the windings and thus continuous variation of the distance D and thus of the length L. One or more hooks, protrusions, roughened portions, undulations, etc. along the windings may provide predetermined attachment positions for associated lengths L.

In use, the coupling means 3 are attached and fixed to the spring 2 at a desired position, e.g. D2, to provide a desired length L, e.g. L2. Next, at least a portion of the users' body part, here a penis, is inserted into the spring 2 so that it is received therein and the proximal end 4 of the spring 2 is arranged against the body, e.g. the pelvic bone. The spring 2 is compressed and the coupling means 6 are fixed about the penis, e.g. by tying a knot in the strap or fastening a clasp, known per se. Different sizes of penises can thus be accommodated. Then the spring 2 is (gently) released and the spring tension is applied to the penis for any desired period of time. During successful treatment the penis extends and the length L of the device 1 may be extended to maintain a constant tension.

A spring, in particular a coiled spring, is generally flexible and can provide a significant force at little weight. Thus, the device allows following a users motion with little interference, allows following size changes of the penis, e.g. due to temperature variations and/or erections, and may be worn inconspicuously. The device 1 may therefore be worn for prolonged periods and in regular daily life providing extensive treatment.

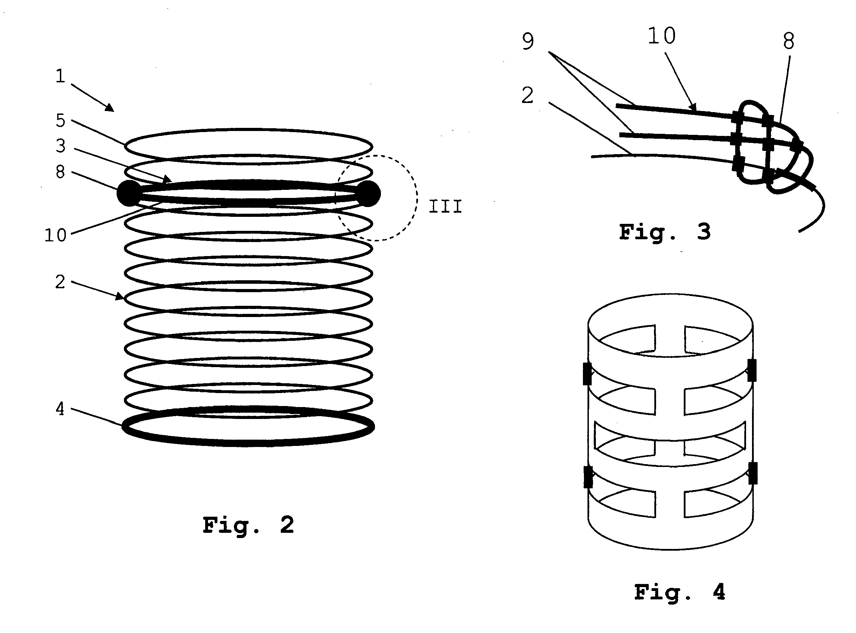

A particularly simple and effective embodiment is shown in Fig. 2 with detail III shown in Fig. 3. In this embodiment, the coupling means 3 comprise adjacent portions 9 of one or more elastic bands 10 tied to the spring 2 with knots 8, here a single band 10. The distal portion of the penis is clamped between the adjacent portions 9 of the band 10. The knots 8 allow repositioning of the band 3 along the spring 2 for adjusting the effective length and tension of the device, e.g. by rotating the band 3 and the spring 2 with respect to each other in "screwing" fashion. The elastic band 10 may be an O-ring, looped about a winding of the spring 2 as shown in Fig. 3. Such looped knot 8 automatically pulls taut about the spring 2 under tension and thus increases reliability of the position of the coupling means 3 with respect to the spring 2 and thus the length and effective spring force of the device 1. The elasticity of the portions 9 of the band 10 may determine a proper holding force onto the penis, e.g. for accommodating different penis sizes and girths which are expected to vary as a consequence of successful treatment, and it provides security against an excessive pulling force of the device 1, allowing the penis to slip out of the coupling.

The device 1 provides a pulling force substantially along the axis of extension of the spring 2 and thus prevents undesired deformation and/or asymmetry. Flexibility of the device 1 in sideways direction following natural movement and/or the cut of clothing, facilitating use and wearing the device.

Since the spring 2 of the device 1 may be cylindrical, i.e. having a substantially constant diameter as shown in the embodiments, the spring constant may be constant throughout the length of the spring 2, allowing a reliable adjustment of the length and spring tension of the device 1. Further, the device may be manufactured cost efficient using a standard product.

The coupling means, or mounting portion, may comprise or be made of rubber, latex, etc, and the spring may be a plastics material or a metal such as surgical steel or similar (combinations of) materials. One or more portions of the device may be coated and/or coloured. Preferably the materials are washable. The device may be worn to the naked skin or to clothed or bandaged skin or, in particular for a penis extender, with a plastics or other layer in between e.g. a condom.

The invention is not restricted to the above described embodiments which can be varied in a number of ways within the scope of the claims. For instance, instead of a wound coil-type spring, the spring may be formed by a tube with a wall with periodical radial cuts along the tubes direction of extension, as indicated in Fig. 4, or having a harmonica-wall with oscillating diameter along the direction of extension so as to impart flexibility and resiliency to the tube.

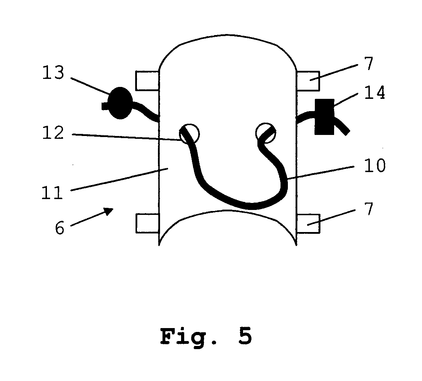

Instead of an elastic O-ring a band may be provided. As shown in Fig. 5, the coupling piece 6 may further comprise one or more guiding pieces 11, e.g. a half-tube or cup-like, which may spread out the otherwise rather local force of a band or ribbon 10 on the body part to be extended. The band 10 may be coupled to the piece 11 through holes 12 and with one or more knots 13 and/or clips 14. A clip 14 is which preferably releasably and/or movably attachable or attached to the band 10, e.g. being a spring-loaded clasp, for adjusting the length of the band 10 and therewith for adjusting the holding force of the coupling means 3 on the body part. The mounting portion may also comprise one or more clips for clamping to the body part to be extended; the mounting portion or clips may be provided with padding means to prevent excessive pinching. Similarly, a band 10 may comprise one or more widened portions.

Alternatively, the device can provide a compression force to the body part to shorten it, in particular when the proximal portion of the spring is further provided with coupling means for attaching to the further body part. Such coupling means may comprise a belt.

Elements and aspects shown and/or discussed for or in relation with a particular embodiment may be suitably combined with elements and aspects of other embodiments, unless explicitly stated otherwise.

## Claims

1. Device (1) for shaping a body part comprising
a spring (2) and coupling means (3),
the spring being configured for accommodating the body part to be shaped and having a proximal end (4) and a distal end (5),
wherein the proximal end is configured for being arranged at or near a proximal portion of the body part to be shaped and for coupling to and/or abutting a further body part, and the distal end is configured for being arranged at or near a distal portion of the body part to be shaped,
wherein the coupling means are configured for coupling to the distal portion of the body part to be shaped
**characterised in that** the coupling means are attachable to the spring in at least a first position at a first distance (D1) from the proximal end and a second position at a second distance (D2) different from the first distance from the proximal end.

2. Device (1) according to claim 1, wherein the coupling means are releasably and/or movably attachable or attached to the spring.

3. Device (1) according to any preceding claim, wherein the device is configured for accommodating a human penis, with the proximal end (4) abutting a body part at or near the root of the penis and the distal end (5) being arranged at or near the glans penis.

4. Device (1) according to any preceding claim, wherein the spring (2) comprises an axis of extension (A) and the device is flexible in at least one direction perpendicular to the axis of extension.

5. Device (1) according to any preceding claim, wherein the coupling means (3) are movably attached to the spring (2) to be arrangeable in a plurality of positions between the first position and the second position.

6. Device (1) according to any preceding claim, wherein the coupling means (3) are adjustable to accommodate body parts to be shaped of different sizes.

7. Device (1) according to any preceding claim, wherein the coupling means (3) comprise encircling means (6, 9, 10) for effecting encirclement of the portion of the body part to be shaped in a tight-fitting engagement.

8. Device (1) according to any preceding claim, wherein the coupling means (3) comprise one or more elastic bands and/or straps (6, 9, 10).

9. Device (1) according to any preceding claim, wherein the spring (2) comprises a wound coil and the coupling means (3) comprise a loop-shaped elastic band (9, 10).

10. Device (1) according to any preceding claim, wherein the spring (2) comprises an axis of extension (A) and the device is configured to apply a tension force substantially along the axis of extension.

## Patentansprüche

1. Vorrichtung (1) zum Formgeben eines Körperteils, aufweisend eine Feder (2) und Verbindungsmittel (3),
wobei die Feder eingerichtet ist zum Aufnehmen des Körperteils, dem Form zu geben ist, und ein proximales Ende (4) und ein distales Ende (5) hat,
wobei das proximale Ende eingerichtet ist, um an oder nahe einem proximalen Abschnitt des Körperteils, dem Form zu geben ist, angeordnet zu sein und um verbunden zu sein mit und/oder anzuliegen an einem weiteren Körperteil, und wobei das distale Ende eingerichtet ist, um an oder nahe einem distalen Abschnitt des Körperteils, dem Form zu geben ist, angeordnet zu sein,
wobei die Verbindungsmittel eingerichtet sind, um mit dem distalen Abschnitt des Körperteils, dem Form zu geben ist, verbunden zu sein,
**dadurch gekennzeichnet, dass** die Verbindungsmittel an der Feder anbringbar sind in wenigstens einer ersten Position in einem ersten Abstand (D1) von dem proximalen Ende und einer zweiten Position in einem zweiten Abstand (D2), der von dem ersten Abstand verschieden ist, von dem proximalen Ende.

2. Vorrichtung (1) gemäß Anspruch 1, wobei die Verbindungsmittel lösbar und/oder bewegbar an der Feder anbringbar oder angebracht sind.

3. Vorrichtung (1) gemäß irgendeinem vorhergehenden Anspruch, wobei die Vorrichtung eingerichtet ist, um einen menschlichen Penis aufzunehmen, wobei das proximale Ende (4) an einem Körperteil an oder nahe der Basis des Penis anliegt und das distale Ende (5) an oder nahe der Peniseichel angeordnet ist.

4. Vorrichtung (1) gemäß irgendeinem vorhergehenden Anspruch, wobei die Feder (2) eine Extensionsachse (A) hat und die Vorrichtung in wenigstens einer Richtung senkrecht zu der Extensionsachse (A) flexibel ist.

5. Vorrichtung (1) gemäß irgendeinem vorhergehenden Anspruch, wobei die Verbindungsmittel (3) an der Feder (2) bewegbar angebracht sind, um in einer Mehrzahl von Positionen zwischen der ersten Position und der zweiten Position angeordnet werden zu können.

6. Vorrichtung (1) gemäß irgendeinem vorhergehenden Anspruch, wobei die Verbindungsmittel (3) einstellbar sind, um Körperteile, denen Form zu geben ist, unterschiedlicher Größe aufzunehmen.

7. Vorrichtung (1) gemäß irgendeinem vorhergehenden Anspruch, wobei die Verbindungsmittel (3) Ringmittel (6, 9, 10) aufweisen zum effektiven Umgeben des Abschnitts des Körperteils, dem Form zu geben ist, in einem Eng-Anliege-Eingriff.

8. Vorrichtung (1) gemäß irgendeinem vorhergehenden Anspruch, wobei die Verbindungsmittel (3) ein oder mehrere elastische Bänder und/oder Streifen (6, 9, 10) aufweisen.

9. Vorrichtung (1) gemäß irgendeinem vorhergehenden Anspruch, wobei die Feder (2) eine gewickelte Spule aufweist und die Verbindungsmittel (3) ein schleifenförmiges Elastikband (9, 10) aufweisen.

10. Vorrichtung (1) gemäß irgendeinem vorhergehenden Anspruch, wobei die Feder (2) eine Extensionsachse (A) hat und die Vorrichtung eingerichtet ist, um eine Spannkraft im Wesentlichen entlang der Extensionsachse (A) auszuüben.

## Revendications

1. Dispositif (1) pour mettre en forme une partie de corps, comprenant un ressort (2) et des moyens d'accouplement (3),
le ressort étant configuré pour loger la partie de corps destinée à être mise en forme et comportant une extrémité proximale (4) et une extrémité distale (5),
dans lequel l'extrémité proximale est configurée pour être agencée à une, ou près d'une, portion proximale de la partie de corps destinée à être mise en forme est pour s'accoupler à et/ou venir porter sur une partie de corps supplémentaire, et l'extrémité distale est configurée pour être agencée à une, ou près d'une, portion distale de la partie de corps destinée à être mise en forme,
dans lequel les moyens d'accouplement sont configurés pour s'accoupler à la portion distale de la partie de corps destinée à être mise en forme,
**caractérisée en ce que** les moyens d'accouplement sont fixables au ressort dans au moins une première position à une première distance (D1) de l'extrémité proximale et une seconde position à une seconde distance (D2), différente de la première distance, de l'extrémité proximale.

2. Dispositif (1) selon la revendication 1, dans lequel les moyens d'accouplement sont fixables ou fixés au ressort de façon détachable et/ou de façon mobile.

3. Dispositif (1) selon une quelconque revendication précédente, dans lequel le dispositif est configuré pour loger un pénis humain, avec l'extrémité proximale (4) venant porter sur une partie de corps à, ou près de, la base du pénis et l'extrémité distale (5) agencée, ou près du, gland.

4. Dispositif (1) selon une quelconque revendication précédente, dans lequel le ressort (2) comprend un axe d'extension (A) et le dispositif est flexible dans au moins une direction perpendiculaire à l'axe d'extension.

5. Dispositif (1) selon une quelconque revendication précédente, dans lequel les moyens d'accouplement (3) sont fixés au ressort (2) de façon mobile pour pouvoir être agencés dans une pluralité de positions entre la première position et la seconde position.

6. Dispositif (1) selon une quelconque revendication précédente, dans lequel les moyens d'accouplement (3) sont réglables pour loger des parties de corpus, destinées à être mises en force, de tailles différentes.

7. Dispositif (1) selon une quelconque revendication précédente, dans lequel les moyens d'accouplement (3) comprennent des moyens d'encerclement (6, 9, 10) pour effectuer un encerclement de la portion de la partie de corps, destinée à être mise en forme, en prise à ajustement serré.

8. Dispositif (1) selon une quelconque revendication précédente, dans lequel les moyens d'accouplement (3) comprennent une ou plusieurs bandes et/ou sangles élastiques (6, 9, 10).

9. Dispositif (1) selon une quelconque revendication précédente, dans lequel le ressort (2) comprend une spire enroulée et les moyens d'accouplement (3) comprennent une bande élastique en forme de boucle (9, 10).

10. Dispositif (1) selon une quelconque revendication précédente, dans lequel le ressort (2) comprend un axe d'extension (A) et le dispositif est configuré pour appliquer une force de tension sensiblement le long de l'axe d'extension.
